(19) Europäisches Patentamt
European Patent Office
Office européen des brevets

(11) **EP 0 991 666 B1**

(12) **EUROPÄISCHE PATENTSCHRIFT**

(45) Veröffentlichungstag und Bekanntmachung des Hinweises auf die Patenterteilung:
**28.11.2007 Patentblatt 2007/48**

(21) Anmeldenummer: **98921263.4**

(22) Anmeldetag: **20.05.1998**

(51) Int Cl.:
*C07K 14/81* (2006.01)    *A61K 38/57* (2006.01)
*C07K 1/16* (2006.01)

(86) Internationale Anmeldenummer:
**PCT/AT1998/000130**

(87) Internationale Veröffentlichungsnummer:
**WO 1998/056821 (17.12.1998 Gazette 1998/50)**

(54) **ALPHA 1-ANTITRYPSIN-PRÄPARATION SOWIE VERFAHREN ZU DEREN HERSTELLUNG**

ALPHA 1-ANTITRYPSIN PREPARATION AND METHOD FOR THE PRODUCTION THEREOF

PREPARATION D'ALPHA 1-ANTITRYPSINE ET SON PROCEDE DE FABRICATION

(84) Benannte Vertragsstaaten:
**AT BE CH CY DE DK ES FI FR GB GR IE IT LI LU MC NL PT SE**
Benannte Erstreckungsstaaten:
**SI**

(30) Priorität: **10.06.1997 AT 100797**

(43) Veröffentlichungstag der Anmeldung:
**12.04.2000 Patentblatt 2000/15**

(73) Patentinhaber: **Baxter Aktiengesellschaft**
**1221 Wien (AT)**

(72) Erfinder:
• **MATTES, Erwin**
**A-2380 Perchtoldsdorf (AT)**
• **MATTHIESSEN, H., Peter**
**A-1020 Wien (AT)**

(74) Vertreter: **Alge, Daniel et al**
**Sonn & Partner Patentanwälte**
**Riemergasse 14**
**1010 Wien (AT)**

(56) Entgegenhaltungen:
**EP-A- 0 097 274          EP-A- 0 221 426**
**EP-A- 0 698 615          WO-A-95/35306**

• **S.K.CHAN ET AL.: "PURIFICATION AND CHEMICAL COMPOSITIONS OF HUMAN ALPHA 1-ANTITRYPSIN OF THE MM TYPE" FEBS LETTERS, Bd. 35, Nr. 1, September 1973, Seiten 79-82, XP002075411 AMSTERDAM NL**
• **V.Y. BASIS ET AL.: "PURIFICATION OF HUMAN ALPHA-1-INHIBITOR OF PROTEASES AND PRODUCTION OF ANTISERUM FOR THE PROTEIN IMMUNOCHEMICAL ESTIMATION." VOPROSY MEDICINSKOJ CHIMII, Bd. 33, Nr. 1, 1987, Seiten 54-59, XP002075412 MOSCOW, RU in der Anmeldung erwähnt**
• **C.B. GLASER ET AL.: "THE ISOLATION OF ALPHA-1-PROTEASE INHIBITOR BY A UNIQUE PROCEDURE DESIGNED FOR INDUSTRIAL APPLICATION." ANALYTICAL BIOCHEMISTRY., Bd. 124, 1982, Seiten 364-371, XP002075413 NEW YORK US**

EP 0 991 666 B1

**Beschreibung**

Die Erfindung betrifft Alpha 1-Antitrypsin-Präparationen, Verfahren zu deren Herstellung sowie Verwendungen dieser Präparationen.

Alpha-1-Antitrypsin ($\alpha$1-AT) ist ein in Plasma vorkommendes Protein, welches auf Grund seiner strukturellen und funktionellen Eigenschaften in die Superfamilie der Serpine eingeordnet wird (<u>Serine</u> <u>p</u>rotease <u>in</u>hibitors). Auf Grund seiner Serinprotease inhibierenden Wirkung ist $\alpha$1-AT auch unter dem Namen $\alpha$1-Proteinase-Inhibitor bekannt. $\alpha$1-AT ist für ungefähr 90 % der tryptischen Inhibitionskapazität von Normalplasma verantwortlich, weshalb es oft auch als Haupt-Plasma-Serpin (Major plasma serpin) bezeichnet wird. Von besonderer physiologischer Bedeutung ist die inhibitorische Aktivität des $\alpha$1-AT bezüglich der Elastase.

$\alpha$1-AT ist primär ein Schutzprotein, wodurch Zellen von freigesetzten proteolytischen Enzymen geschützt werden sollen. Es wird in der Leber synthetisiert und ins Plasma sekretiert, worin es eine Halbwertszeit von ungefähr 6 Tagen hat. Die normale Plasmakonzentration von $\alpha$1-AT beträgt 1,3 g/l.

$\alpha$1-AT ist ein relativ kleines und polares Molekül, welches schnell in die Zellflüssigkeiten gelangt und dort wirken kann. Menschliches $\alpha$1-AT besteht aus einer einzigen Polypeptidkette mit 394 Aminosäureresten mit drei Glykosylierungspositionen an den Asparaginresten der Positionen 46, 83 und 247. Während an Asparagin 46 und 247 sowohl zwei- als auch drei-antennige sialysierte Kohlehydrat-Seitenketten vorhanden sind, ist an Asparagin 83 nur eine sialysierte bi-antennige Seitenkette vorhanden (Carrell et al., Nature 298 (1982), 329-334).

Die Proteinkette von $\alpha$1-AT kommt in zwei Formen im Plasma vor, wobei in der einen Form ein N-terminales Pentapeptid entfernt ist.

Aufgrund dieser Kohlehydrat-Seitenketten, aber auch aufgrund der möglichen Pentapeptid-Entfernung kommt es zu einer recht ausgeprägten Mikroheterogenität, welche sich in ein und demselben Individuum auch ändern kann. So kommt es vor, daß erhebliche Änderungen in den Proportionen dieser Isoformen der $\alpha$1-AT-Moleküle während Entzündungen oder Östrogenverabreichungen vorkommen, was wahrscheinlich darauf zurückzuführen ist, daß aufgrund der Streßsituation die teilweise desialysierten Formen bevorzugter abgebaut werden, um schneller wieder zu normalen Plasma-Levels zu kommen (Patterson, Comp. Biochem. Physiol. 100 B (3) (1991), 439-454).

Die Serinprotease inhibierende Wirkung von $\alpha$1-AT ist auf die Formation von stabilen 1:1-Komplexen zwischen $\alpha$1-AT und der Zielprotease zurückzuführen. $\alpha$1-AT wirkt daher als eine Art "Suizidsubstrat", mit welcher die weitere Proteasereaktion der Serinprotease unterbunden wird. Die Wirkung von $\alpha$1-AT wird vor allem von freigesetzten Radikalen inhibiert, die ebenfalls im Zuge von Entzündungen entstehen. Physiologisch dient diese Oxidationslabilität wahrscheinlich dazu, in der unmittelbaren Umgebung der Entzündung die Enzym-inhibierende Aktivität von $\alpha$1-AT zu unterbinden, so daß Proteasen, wie Elastase und Kathepsin G ihre volle Wirkung bei der Bekämpfung beispielsweise entzündungsauslösender Bakterien entfalten können.

Die Oxidationslabilität von $\alpha$1-AT ist jedoch vor allem dort nachteilig, wo das $\alpha$1-AT bzw. die Flüssigkeit, die $\alpha$1-AT enthält, exponiert wird, wie z.B. in den Flüssigkeiten des Respirationstraktes. So wird das elastische Gewebe der Lunge vor Oberflächenangriffen im wesentlichen von zwei Inhibitoren geschützt, dem humanen sekretorischen Leukozyten-Proteaseinhibitor, der hauptsächlich im oberen respiratorischen Trakt zu finden ist, und $\alpha$1-AT, welches vorwiegend im unteren respiratorischen Trakt vorkommt. Obwohl normalerweise mehr als genug inhibitorische Kapazität für die Verteidigung des unteren Respirationstraktes vorhanden ist, kann es bei übermäßiger Exposition an freie Radikale, wie z.B. bei starkem Rauchen, zu Problemen kommen (die inhibitorische Kapazität von $\alpha$1-AT ist bei starken Rauchern ungefähr halbiert).

Mittlerweile sind über 70 qualitative und quantitative Varianten von menschlichem $\alpha$1-AT bekannt, die als autosomal co-dominante Allele vererbt werden, wobei davon ausgegangen wird, daß ungefähr 10 % (!) der europäischen Bevölkerung Träger einer pathologischen Variante von $\alpha$1-AT sind. Auch $\alpha$1-AT-Defizienzen sind bekannt und relativ weit verbreitet. Die bemerkenswertesten pathologischen Befunde im Zusammenhang mit der $\alpha$1-AT-Genvarianz sind eine relativ früh beginnende degenerative Lungenerkrankung sowie eine schwere Lebererkrankung. Daneben werden aber auch Nierenerkrankungen, Arthritis und maligne Erkrankungen mit einer $\alpha$1-AT-Genvarianz in Verbindung gebracht. Vor allem bei der Lungenerkrankung ist sicher, daß diese direkt auf den jeweiligen Plasmalevel von $\alpha$1-AT zurückzuführen ist, welcher wegen der kumulierten proteolytischen Beschädigung einen Verlust der Lungenelastizität bewirkt. So ist ein starker Raucher mit einem homozygoten $\alpha$1-AT-Mangel doppelt gefährdet und hat ein hohes Risiko, eine schwere Emphysemie bereits im Alter von unter 40 Jahren zu entwickeln. Gegenüber dem nichtrauchenden Homozygoten hatte er eine um 20 Jahre verkürzte Überlebenszeit.

In Plasma kommt das $\alpha$1-AT sowohl in aktiver als auch in inaktiver Form vor (siehe beispielsweise Pajdak W. et al., Folia Histochemica et Cytobiologica, Vol.24 (1986), S. 169-172).

Es sind eine Reihe von Herstellungsverfahren für $\alpha$1-AT bekannt, welche die fraktionierte Fällung von Plasma mit Polyethylenglykol 4000, aber auch die Aufbereitung von verschiedenen Plasmafraktionen (Cohn-Fraktion IV-1-Präzipitat oder Kistler und Nitschmann-Überstand A oder A+) umfaßt (Feldman und Winkelman, Blood Separation and Plasma Fractionation (1991), Wiley-Liss, Inc., S. 341-383).

Gemäß der WO 95/35306 A wird α1-AT durch PEG-Fällung, Behandlung mittels Annionenaustauscher und Metallchelat-Medium aufgereinigt.

Bei weitergehenden Reinigungen wurden die betreffenden Blutfraktionen beispielsweise mit DEAE-Zellulose gereinigt (Basis et al. (Vopr. Med. Khim. 33 (1) (1987), 54-59); Chan et al. (FEBS Lett. 35(1) (1973), 79-82)), mit Affinitätschromatographie-Materialien oder mit Kationenaustauscher-Chromatographie-Materialien (EP-0 698 615-A1) behandelt.

Basis et al. beschreiben ein Verfahren zur Aufreinigung von α1-AT durch Ammoniumsulfatfällung von Plasma und anschließender DEAE-Zellulose-Chromatographie und Hydroxylapatit-Chromatographie. Dieses Verfahren wurde ständig in Gegenwart von Mercaptoethanol, welches das Protein gegenüber Oxidation S-hältiger Gruppen schützen soll, vorgenommen. α1-AT wird nach der Hydroxylapatit-Chromatographie in zwei Fraktionen gewonnen, je doch werden die beiden Proteine, α1-AT und Albumin, nicht voll ständig aufgetrennt.

Im Zuge der Vorarbeiten zur vorliegenden Erfindung wurde nun festgestellt, daß es mit allen herkömmlichen Herstellungsverfahren, insbesondere aber auch bei den Verfahren gemäß Basis et al. und gemäß der EP-0 698 615-A1, nicht gelungen ist, das konstitutiv in Plasma mit dem aktiven α1-AT vorkommende inaktive α1-AT vollständig abzutrennen bzw. eine Präparation unter Erhalt aktiver Isomere herzustellen.

Aufgabe der vorliegenden Erfindung ist daher, ein Verfahren zur Verfügung zu stellen, mit welchem eine α1-AT-Präparation gewonnen werden kann, in der das Verhältnis von aktivem α1-AT zu in aktivem α1-AT zugunsten von aktivem α1-AT verbessert wird, d.h., mit welchem inaktives α1-AT selektiv von aktivem α1-AT abgetrennt werden kann.

Eine weitere Aufgabe der vorliegenden Erfindung besteht darin, eine gegenüber den herkömmlichen Präparaten verbesserte ?1-AT-Präparation zur Verfügung zu stellen.

Den Gegenstand der vorliegenden Erfindung bildet daher eine Präparation auf Basis von nativem, chromatographisch gereinigtem α1-AT, welche wenigstens das Isomer mit einem pI-Wert zwischen 4,3 und 4,4 enthält, eine Reinheit von wenigstens 1,0 PE/mg Protein und eine relative Plasma-α1-AT-Aktivität von mindestens 120 % aufweist.

Die relative Plasma-α1-AT-Aktivität ist definiert als das Verhältnis von aktivem zu inaktivem α1-AT, wobei dieses Verhältnis in Plasma mit 100 % relativer Plasma-α1-AT-Aktivität angenommen wird. Für die erfindungsgemäß zur Verfügung gestellten Präparationen liegt diese relative Plasma-α1-AT-Aktivität vorzugsweise über 130 %, noch bevorzugter über 140 %, insbesondere über 150 %. In besonderen Fällen liegt die relative Plasma-α1-AT-Aktivität auch über 160 %. Bei den erfindungsgemäßen Produkten ist aufgrund deren Reinheit das enthaltene Protein annähernd mit dem α1-AT-Protein gleichzusetzen, wodurch die relative Plasma-α1-AT-Aktivität in einfacher Weise zu berechnen ist.

α1-AT liegt in Blut bzw. Serum zu einem gewissen Anteil immer auch in inaktiver Form vor, d.h. es besitzt praktisch keine Elastase hemmende Aktivität, ist aber noch immunreaktiv. Die Gesamtmenge an (aktivem und inaktivem) α1-AT kann mit allen gängigen Methoden bestimmt werden, z.B. Antigen-quantifizierende immunologische Methoden, wie ELISA.

Die Ursachen für das Vorliegen von inaktivem α1-AT können mannigfaltig sein, beispielsweise kann α1-AT aufgrund einer Spaltung des Moleküls oder aufgrund von Störungen in der Konformation inaktiv sein.

Da normalerweise im Blut bzw. Serum bis zu 20 % inaktives α1-AT vorhanden sein können und dieses mit herkömmlichen Methoden nicht abgetrennt werden konnte, stellt die erfindungsgemäße Präparation einen wesentlichen Fortschritt gegenüber herkömmlichen α1-AT-Präparaten, in denen bislang immer der inaktive Anteil "mitgeschleppt" worden war, dar. Wie erwähnt, liegt die Konzentration von α1-AT in Normalplasma bei 1,3 g/l, womit sich eine spezifische Aktivität von 0,77 PE (Plasmaeinheiten, bestimmt im Elastase-Hemmtest, siehe Beispiel 3) /mg ergibt.

Das erfindungsgemäße α1-AT-Präparat enthält vorzugsweise wenigstens das Isomer mit einem pI-Wert zwischen 4,3 und 4,4.

Es hat sich nämlich überraschenderweise gezeigt, daß gerade aufgrund der Anwesenheit dieses Isomers die Aktivität des α1-AT sehr hoch ist. Da das Isomer mit einem pI-Wert zwischen 4,3 und 4,4 aufgrund seiner aziden Natur in den im Stand der Technik beschriebenen Verfahren zur Gewinnung von α1-AT immer zusammen mit Albumin von der zu gewinnenden α1-AT-Fraktion abgetrennt worden ist, finden sich in keinem α1-AT-Präparat bislang nennenswerte Anteile an diesem Isomer. Im Zuge der vorliegenden Erfindung wurde jedoch die Relevanz gerade dieses Isomers bezüglich der Aktivitätsausbeute erkannt und es werden daher erfindungsgemäß im Herstellungsverfahren bevorzugterweise nur Schritte vorgesehen, die eine Abtrennung dieses Isomers vom übrigen α1-AT nicht oder nur zu einem unwesentlichen Teil erlauben, sodaß auch der Großteil dieses sauren Isomers im Endpräparat vorhanden ist.

Die Isomerenverteilung der bevorzugten Präparation entspricht also der des nativen Proteins, insbesondere dem Protein, das aus einem Plasmapool erhältlich ist. Insbesondere entspricht die Isomerenverteilung der erfindungsgemäßen α1-AT-Präparation bei Sichtbarmachung durch isoelektrische Fokussierung (IEF) einem Quadruplett-Bandenmuster.

Die IEF wird insbesondere mit dem von Pharmacia erhältlichen Gel Ampholine PAG plate IEF™ (pH 4,0 - 6,5 oder pH 4,0 - 5,0) entsprechend der von Pharmacia gegebenen Vorschrift unter Verwendung von IEF-Markern der Firma Sigma (IEF Mix 3,6 - 6,6) durch geführt.

Das Isomer mit einem pI-Wert zwischen 4,3 und 4,4 entspricht da bei der sauersten Bande. Dieses Isomer fehlt aber gerade bei reinen bzw. hochreinen α1-AT-Präparationen gänzlich bzw. ist es nur in einem geringen Ausmaß unterhalb der Nachweisgrenze enthalten.

Die erfindungsgemäße α1-AT-Präparation weist insbesondere eine hohe Reinheit auf, beispielsweise eine Reinheit von mehr als 1 PE/mg. Es hat sich sogar gezeigt, daß erfindungsgemäß Reinheiten von über 1,1 PE/mg, insbesondere von über 1,2 PE/mg, erzielbar sind.

Die erfindungsgemäße Präparation enthält dabei üblicherweise weniger als 10 %, vorzugsweise weniger als 5 %, insbesondere weniger als 2 % inaktives α1-AT.

Es ist gemäß der vorliegenden Erfindung auch möglich, ein α1-AT-Präparat zur Verfügung zu stellen, welches im wesentlichen frei ist von inaktivem α1-AT.

Das Verhältnis von aktivem α1-AT zu inaktivem α1-AT ist in der erfindungsgemäßen Präparation erhöht, insbesondere ist es höher als jenes in humanem Normalplasma. Es ist also gemäß der vorliegenden Erfindung möglich, ein hyperaktives bzw. als naszent zu bezeichnendes α1-AT zu erhalten.

Unter Normalplasma ist gemäß der vorliegenden Erfindung ein bezüglich des Gehaltes an aktivem α1-AT standardisiertes Plasma zu verstehen. Die Standardisierung, also die Bestimmung von aktivem α1-AT kann mittels hierfür gängiger Methoden erfolgen. Beispielsweise mittels Hemmung der Elastase- bzw. Trypsinaktivität.

Die erfindungsgemäße Präparation hat dabei gegenüber den Präparationen gemäß dem Stand der Technik den wesentlichen Vorteil, daß das insbesondere zur pharmazeutischen Verabreichung vorgesehene Präparat durch den niedrigeren Gehalt an inaktivem α1-AT hinsichtlich seiner Wirksamkeit sehr viel definierter ist als die bekannten Präparate, was besonders bei medizinischer Verwendung von enormen Vorteil ist.

Bevorzugterweise wird ein konservierendes α1-AT zur Verfügung gestellt, bei welchem die Verwendung von Oxidationsstabilisatoren, wie β-Mercaptoethanol, vermieden werden kann. "Konservierend" bedeutet daher im Rahmen der vorliegenden Erfindung, daß das α1-AT auch ohne die Anwesenheit von Oxidationsstabilisatoren, wie β-Mercaptoethanol, ausreichend stabil ist, was insbesondere angesichts der bekannten großen Oxidationslabilität von α1-AT besonders überraschend war.

Die erfindungsgemäße Präparation kann ausgehend von Blut, Plasma, Serum, oder Fraktionen derselben, aber auch ausgehend von Zellkulturen, insbesondere rekombinanten Zellkulturen, oder Zellkulturüberständen hergestellt werden.

Bevorzugterweise ist der Monomergehalt der erfindungsgemäßen Präparation mindestens 95 %, vorzugsweise mindestens 98 %.

Eine bevorzugte Ausführungsform des erfindungsgemäßen Präparates besteht darin, dass die Präparation nicht nur hinsichtlich des Gehaltes an aktivem α1-AT verbessert ist, sondern dass sie auch in hoher Reinheit vorliegt, beispielsweise von mindestens 90 % (mg/mg Protein).

Gegenstand der vorliegenden Erfindung ist auch eine pharmazeutische Präparation, enthaltend die erfindungsgemäße α1-AT-Präparation, gegebenenfalls mit pharmazeutisch annehmbaren Hilfssubstanzen, wie Puffer, Stabilisatoren, Adjuvantien, Antioxidantien, Salzen oder Exzipienten.

Da die vorliegende Präparation hauptsächlich im pharmazeutischen Bereich eingesetzt werden soll, besteht eine bevorzugte Ausführungsform darin, dass die Präparation zur Inaktivierung gegebenenfalls vorhandener Pathogene behandelt ist. Günstigerweise wird die Präparation in einer laberstabilen Form zur Verfügung gestellt, vorzugsweise als Lyophilisat oder als (tiefgefrorene) Lösung, insbesondere als eine Lösung, welche zur i.v.-Verabreichung, als Aerosol oder als Spray geeignet ist. Die Präparation kann auch mit Liposomen bzw. Phospholipiden oder anderen mikro- oder nanopartikulären Formen assoziiert vorliegen, was bei bestimmten Applikationsformen vorteilhaft ist.

Gegenstand der vorliegenden Erfindung ist ebenfalls ein Verfahren zur Herstellung des erfindungsgemäßen α1-AT-Präparates durch Reinigung einer α1-AT enthaltenden Fraktion, welche vorzugsweise aus einem humanen Plasmapool mittels Adsorptionschromatographie derart erhältlich ist, dass α1-AT adsorbiert, gegebenenfalls inaktives α1-AT abgetrennt wird und aktives α1-AT, enthaltend das Isomer mit einem pI-Wert zwischen 4,3 und 4,4, in einer Fraktion durch Eluieren gewonnen wird.

Unter Adsorptionschromatographie ist gemäß der vorliegenden Erfindung eine solche Chromatographie zu verstehen, bei der das α1-AT an das Chromatographiematerial adsorbiert (gebunden) wird. Schließlich kann durch Elution die gewünschte Fraktion gewonnen werden.

Für die Adsorptionschromatographie kann beispielsweise ein anorganisches Chromatographiematerial, wie z.B. Hydroxylapatit, bevorzugterweise keramisches Hydroxylapatit, eingesetzt werden. Die Adsorptionschromatographie kann aber auch an einem Anionenaustauscher, bevorzugterweise in Gegenwart eines Detergens, durchgeführt werden.

Als Anionenaustauscher werden bevorzugterweise Materialien auf Basis von Kohlenhydraten oder Vinylpolymeren eingesetzt, die sich in der Laborarbeit und industriellen Anwendung bewährt haben, insbesondere kommerziell erhältliche Produkte, wie beispielsweise DEAE-Sephacel®, DEAE-Sephadex®, DEAE-Sepharose® CL6B, DEAE-Sepharose® Fast Flow, QAS-Sephadex®, Q-Sepharose® Fast Flow, Q-Sepharose Big Beats®, Q-Sepharose® High Performance (Fa. Pharmacia); DEAE-Trisacryl, DEAE-Spherodex®, Q-Hyper-D® (Fa. Sepracor); DEAE-Toyopearl®, QAE-Toyopearl®, Toyopearl Super-Q® (Fa. Tosohaas); Fractogel®-EMDT, -TMAE oder andere Fractogelmaterialien, Licrospher 1000 TMAE®, Licrospher 1000 DEAE® und Licrospher 4000 DMAE® (Fa. Merck); Macroprep DEAE®, Macroprep Q® (Fa. BioRad); Protein PAK DEAE® (Fa. Waters), wobei die Behandlung mit einem starken Anionenaustauscher, z.B. Q-Sepharose besonders bevorzugt ist. Die genauen Bedingungen, bei welchen die Gewinnung von aktivem α1-AT erfolgt, können je nach Austauschermaterial schwanken; diese sind aber für den Fachmann für das jeweilige Material in Kenntnis der vorliegenden Erfindung leicht und ohne großen Aufwand zu ermitteln bzw. zu optimieren.

Bevorzugterweise wird das Ausgangsmaterial bei bzw. vor der Adsorptionschromatographie nicht bzw. nur für sehr kurze Zeit (z.B. wenige Minuten) inkubiert. Die Adsorptionschromatographie bzw. Gewinnung des α1-AT erfolgt insbesondere unter solchen Bedingungen, daß das Isomer mit einem pI-Wert zwischen 4,30 und 4,40, insbesondere zwischen 4,34 und 4,39, erhalten bleibt, und besonders bevorzugt derart, daß das Spektrum aktiver Isomere auch während bzw. nach der Reinigung bzw. Behandlung erhalten bleibt. Diese Bedingungen sind durch Ausprobieren von verschiedenen Elutionspuffern für die jeweils verwendeten Adsorptionsmaterialien für den Fachmann leicht optimierbar, da das Isomer mit einem pI-Wert zwischen 4,3 und 4,4 bzw. Fraktion, in der dieses Isomer enthalten ist, leicht durch IEF delektierbar ist. Hierbei muß insbesondere darauf geachtet werden, daß dieses saure Isomer nicht mit Albumin abgetrennt wird. Dies ist für den Fachmann jedoch ohne weiteres, z.B. mit Gelelektrophorese bzw. IEF-Überprüfung der eluierten Fraktionen (und der Detektion von saurem α1-AT und Albumin), möglich.

In einer weiteren bevorzugten Ausführungsform wird keine Chromatographie an einem Kationenaustauscher durchgeführt, insbesondere keine Kationenaustauscher-Chromatographie bei niedrigem pH-Wert.

Unter einem Detergens (Tensid) ist üblicherweise eine grenzflächenaktive organische Substanz zu verstehen, insbesondere organische Syntheseprodukte. Erfindungsgemäß werden vorzugsweise nichtionische Detergentien verwendet, wie Polyether, insbesondere Alkyl-, Phenol-, Polyglykolether, Produkte der Ethoxylierung von Fettsäuren, Fettsäureamiden, Fettaminen, Fettalkoholen, Aminoxiden, Fettsäureester von Polyalkoholen und Zuckerestern. Das Detergens (Tensid) wirkt insbesondere auf Proteine nicht denaturierend. Besonders bevorzugt ist für die Zwecke der vorliegenden Erfindung ein Tensid aus der Gruppe der Polysorbate (z.B. Tween) und ein Tensid aus der Triton®-Gruppe.

Der Adsorptionschromatographie kann beim erfindungsgemäßen Verfahren gemäß einer bevorzugten Ausführungsform eine weitere Behandlung, wie z.B. eine Fällung, Filtration, Gelfiltration, Behandlung mit einem anorganischen Trägermaterial oder eine chromatographische Reinigung vor- oder nachgeschaltet sein. Als bevorzugtes anorganisches Trägermaterial hat sich dabei Hydroxylapatit herausgestellt, wobei keramisches Hydroxylapatit besonders bevorzugt wird. In einer weiteren bevorzugten Ausführungsform wird die Adsorptionschromatographie an einem Anionenaustauscher, vorzugsweise in Gegenwart eines Detergens, mit einer Adsorption an Hydroxylapatit kombiniert.

Im Zuge der Arbeiten zur vorliegenden Erfindung hat sich überraschenderweise herausgestellt, daß es unter besonderen Umständen möglich ist, auch durch die Behandlung mit anorganischen Materialien, wie Hydroxylapatit, eine Trennung von aktivem α1-AT von inaktivem α1-AT herbeizuführen. Dieser Effekt tritt dann besonders hervor, wenn das α1-AT bereits wesentlich von Albumin abgetrennt ist.

Daher ist auch die Behandlung einer vorgereinigten α1-AT-Präparation mit dem anorganischen Trägermaterial, insbesondere mit Hydroxylapatit, alleine geeignet, eine erfindungsgemäße Präparation herzustellen. Bevorzugterweise wurde aus dem Ausgangsmaterial Albumin bereits vorher zu einem hohen Prozentsatz entfernt. Die Abtrennung des aktiven α1-AT erfolgt bevorzugterweise durch fraktionierte Elution. Die Behandlung mit Hydroxylapatit stellt bevorzugterweise den terminalen Reinigungsschritt dar.

Es hat sich überraschenderweise gezeigt, daß, obwohl α1-AT bekanntermaßen extrem oxidationslabil ist, das erfindungsgemäße Verfahren auch ohne Anwendung von β-Mercaptoethanol oder anderen Oxidationshemmern durchgeführt werden kann, die speziell bei der Herstellung von pharmazeutischen Präparaten zu vermeiden sind.

Das erfindungsgemäße Verfahren wird daher bevorzugterweise in Abwesenheit von β-Mercaptoethanol oder anderen Oxidationsstabilisatoren durchgeführt, d.h., es werden keine Puffer verwendet, die einen derartigen Stabilisator enthalten.

Das Ausgangsmaterial stammt bevorzugterweise von einem Plasmapool, insbesondere einem solchen, der sich aus Blutspenden von α1-AT-normalen Individuen ergibt. Für das erfindungsgemäße Verfahren werden bevorzugterweise Plasma oder eine Plasmafraktion verwendet, insbesondere eine an Albumin abgereicherte Ausgangsfraktion, vorzugsweise ein Cohn V-Niederschlag, entsprechend im wesentlichen einer Cohn IV A-Fraktion (gemäß Cohn, Review 28 (1941), 395-417). In einer weiteren bevorzugten Ausführungsform wird von einer vorgereinigten Fraktion ausgegangen, beispielsweise kann von einem kommerziell erhältlichen Präparat, wie Prolastin, ausgegangen werden.

Beim erfindungsgemäßen Verfahren wird auch vorzugsweise ein Schritt zur Inaktivierung bzw. Abreicherung von gegebenenfalls vorhandenen Pathogenen durchgeführt. Diese Inaktivierungs-/Abreicherungsbehandlung wird vorzugsweise durch eine Tensid- und/oder Hitzebehandlung gewährleistet, beispielsweise einer Hitzebehandlung in festem Zustand, insbesondere einer Dampfbehandlung gemäß der EP-0 159 311 oder der EP-0 519 901 oder der EP-0 674 531, aber auch mit organischen Lösungsmitteln und/oder Detergentien, wie z.B. gemäß der EP-0 131 740 und EP-0 050 061.

Weitere Behandlungen zur Inaktivierung von Viren bzw. Pathogenen umfassen auch die Behandlung mit chemischen oder chemisch/physikalischen Methoden, z.B. mit chaotropen Stoffen gemäß der WO 94/13329 oder DE-44 34 538 oder eine Photoinaktivierung.

Eine Bestrahlung oder Nanofiltration stellen bevorzugte physikalische Verfahren zur Abreicherung von Viren im Rahmen der vorliegenden Erfindung dar.

Das erfindungsgemäße Verfahren wird vorzugsweise derart konzipiert, daß dabei auch die Gewinnung von anderen Proteinen möglich ist, insbesondere die Gewinnung von Transferrin, Albumin, Orosomucoid und Apolipoprotein, welche dann in einer weiteren Fraktion der erfindungsgemäßen chromatographischen Behandlungen gewonnen werden können. Die genauen Bedingungen können für jedes Chromatographiematerial von einem Fachmann in Kenntnis der vorliegenden Erfindung leicht ermittelt bzw. optimiert werden.

Der pH-Wert bei der Durchführung des erfindungsgemäßen Verfahrens, insbesondere bei der Elution, liegt vorzugsweise zwischen 5,5 und 8,0, insbesondere um 6,5-6,8. Es hat sich auch gezeigt, daß bei der Durchführung des erfindungsgemäßen Verfahrens die Verwendung eines Puffers mit einer Ionenstärke entsprechend 10 mM an Phosphat günstig ist.

Gemäß einem weiteren Aspekt betrifft die vorliegende Erfindung die Verwendung eines Trägermaterials zur Trennung des inaktiven $\alpha$1-AT von aktivem $\alpha$1-AT. Als festes Trägermaterial zur Adsorption und Trennung wird vorzugsweise ein anorganisches Trägermaterial, wie Hydroxylapatit, verwendet oder ein Anionenaustauscher in Gegenwart von einem Detergens, insbesondere Q-Sepharose in Gegenwart von Tween, in einer weiteren bevorzugten Ausführungsform.

Die vorliegende Erfindung wird an Hand der nachfolgenden Beispiele, auf die sie jedoch nicht beschränkt sein soll, näher erläutert.

**Beispiele** :

**Beispiel 1: Reinigung von $\alpha$1-AT aus Cohn V-Niederschlag** (derzeit nach Ansicht der Anmelderin der beste Weg zur Ausführung der Erfindung)

Der Cohn V-Niederschlag wird mit dem dreifachen Gewicht an A-Puffer (1,2 g/l $Na_2HPO_4$ x 2 $H_2O$ = 6,74 mM, 10 g/l NaCl = 171 mM, pH 7,0 mit 96 % Essigsäure gestellt) 4 bis 6 Stunden bei 4°C durch Rühren suspendiert. Danach wird $\alpha$1-AT aus dieser trüben Suspension mit 17,5 % Ethanol Endkonzentration bei 6°C über Nacht gefällt.

Der abzentrifugierte Niederschlag wird mit dem 24-fachen Gewicht 10 mM Tris-HCl, pH 10,4 (mit 6 M NaOH gestellt) eine ½ Stunde bei Raumtemperatur und anschließend 1,5 Stunden bei 40°C gerührt, danach der pH auf 6,5 gestellt und durch ein CUNO 50SA-Filter geklärt. Dem Filtrat werden 15 % v/v Tween 80 zugesetzt und 2 Stunden bei 26°C gerührt.

Die erhaltene Lösung wird auf eine, mit Q-Sepharose® Fast Flow (Pharmacia) gefüllte und mit 25 mM Natriumphosphatpuffer, pH 6,5, äquilibrierte Chromatographiesäule, entsprechend 10 mg Protein pro ml Gel (etwa 2 Säulenvolumen) aufgetragen, mit 2,5 Säulenvolumen 25 mM Natriumphosphatpuffer, pH 6,5 (eluiert dabei großteils Tween® 80) und weiters mit 3 Säulenvolumen 60 mM Natriumchlorid in 25 mM Natriumphosphatpuffer, pH 6,5 (eluiert Transferrin), gewaschen.

Durch Elution mit 3 Säulenvolumen 100 mM Natriumchlorid in 25 mM Natriumphosphatpuffer, pH 6,5, erhält man die $\alpha$1-AT enthaltende Fraktion mit etwa 0,40 bis 0,60 Plasmaeinheiten (PE) $\alpha$1-AT pro mg Protein, wobei die Aktivität durch Hemmung von Elastase gemäß Beispiel 3 bestimmt wurde (1 PE $\alpha$1-AT entspricht etwa 1,3 mg reinem $\alpha$1-AT).

Mit 3 Säulenvolumen 125 mM Natriumchlorid in 25 mM Natriumphosphatpuffer, pH 6,5, können dann noch hauptsächlich Serumalbumin und weitere Plasmaproteine eluiert werden und mit 1 bis 2 M Natriumchlorid in 25 mM Natriumphosphatpuffer, pH 6,5, inaktives $\alpha$1-AT (praktisch keine Elastasehemmung, aber immunreaktives $\alpha$1-AT) und die restlichen Verunreinigungen.

Die $\alpha$1-AT enthaltende Fraktion wird mit einer Ultrafiltrationsmembran (Ausschlußgrenze 30 000 Dalton) etwa 30-fach konzentriert und mit 0,15 g/l $Na_3$-Citrat x 2 $H_2O$ = 0,5 mM, diafiltriert.

Das erhaltene Diafiltrat wird anschließend lyophilisiert. Das lyophilisierte Pulver wird auf einen Restwassergehalt von 7,5 +/- 0,5 % befeuchtet und der S-TIM 4-Behandlung gemäß der EP-0 159 311 unterzogen (10 Stunden bei 60°C und 1 Stunde bei 80°C).

Das behandelte Pulver wird in 10 mM Natriumphosphatpuffer, pH 6,8, zu etwa 10 mg/ml Protein gelöst und auf eine, mit keramischem Hydroxyapatit (Macropep Typ I, Korngröße 80 $\mu$m, Bio-Rad) gefüllte und mit 10 mM Natriumphosphatpuffer, pH 6,8, äquilibrierte Chromatographiesäule entsprechend 10 mg Protein pro ml Säulenbett (etwa 1 Säulenvolumen), aufgetragen.

Durch Elution mit 5 Säulenvolumen 40 mM Natriumphosphatpuffer, pH 6,8, erhält man die $\alpha$1-AT enthaltende Fraktion mit etwa 0,90 bis 1,05 Plasmaeinheiten (PE) $\alpha$1-AT pro mg Protein.

Mit 3 Säulenvolumen 100 mM Natriumphosphatpuffer, pH 6,8, können dann noch hauptsächlich inaktives $\alpha$1-AT, Serumalbumin und weitere Plasmaproteine eluiert werden und mit 500 mM Natriumphosphatpuffer, pH 6,8, die restlichen Verunreinigungen.

Es zeigte sich, daß mit diesem Reinigungsverfahren α1-AT mit hoher spezifischer Aktivität von 1,0 PE/mg (entspricht 130 % relativer Plasma-α1-AT-Aktivität) erhalten werden kann. Das Ergebnis ist in der nachfolgenden Tabelle 1 dargestellt.

**Tabelle 1**

|  | spez.Aktivität PE/mq | Reinigung | Gesamtausbeute/ % Plasma |
|---|---|---|---|
| Plasma | 0,014 | 1,0 | 100 |
| nach Hydroxyapatit | 1,0 | 70,0 | 14 |

Das am pharmazeutischen Markt befindliche α1-AT-Produkt Prolastin (Fa. Cutter) ist laut Herstellerinformation zwischen 45 - 80 % rein und ergibt im Elastasehemmtest eine spezifische Aktivität von 0,60 PE/mg (entspricht 78 % relativer Plasma-α1-AT-Aktivität).

**Beispiel 2 : Reinigung von α1-AT (Serva) mittels keramischem Hydroxyapatit**

α1-AT von Serva, das mit mindestens 95 % Reinheit (laut Herstellerangaben mittels SDS-Gel bestimmt wurde) die bisher höchste gemessene spezifische Aktivität α1-AT von 0,81 PE/mg aufwies, wurde ebenfalls über keramisches Hydroxyapatit weiterbehandelt. Die vereinigten Fraktionen ergaben eine spezifische Aktivität von 0,92 PE/mg (entspricht etwa 120 % relativer Plama-α1-AT-Aktivität). In dem reduzierenden SDS-Excelgel sieht man mit Silberfärbung der Proteine eine eindeutige Abtrennung von inaktivem α1-AT und anderer verunreinigender Proteine.

**Beispiel 3 : Vorschrift zur Messung der Elastasehemmung**

Der Test wurde in Anlehnung einer Vorschrift von J. Bieth, B. Spiess und C.G. Wermuth (1974), The synthesis and analytical use of a highly sensitive and convenient substrate of elastase, Biochem. Med. 11(4), 350-357, bzw. J. Travis und D. Johnson (1981), Human alpha1-Proteinase Inhibitor, Methods of Enzymology 80, 754-765, erstellt.

Tris:        0,2 M Tris-HCl, pH 8,0

Tris+:       Tris mit 0,1 % humanem Serumalbumin (HSA) kurz vor Verwendung versetzt.

Elastase:    6 % Elastase (aus Schweinepankreas, Boehringer Mannheim) wird 1 : 1000 verdünnt und steril filtriert bei -20°C eingefroren.

Substrat:    22,5 mg Succinyl-(Alanin)$_3$-para-Nitroanilid (Bachem Feinchemikalien AG, Schweiz) wird in 5 ml Dimethyl-formamid aufgelöst und bei 4°C aufbewahrt.

Die Proben werden mit Tris+ bis zur entsprechenden Konzentration verdünnt.

25 μl Elastase werden mit 275 μl Tris+ (im Wasserbad auf 25°C temperiert) verdünnt und mit 100 μl Probe 3 Minuten bei 25°C inkubiert. Danach wird das Substrat 1 : 10 mit Tris+ verdünnt, 200 μl davon zu dem Ansatz gemischt und sofort die Extinktionszunahme bei 405 nm und 25°C über 5 Minuten lang gemessen. Als Leerwert der Hemmung werden statt einer Probe 100 μl Tris+ zum Ansatz gemischt.

Der Leerwert ergibt bei obigem Ansatz zwischen 0,05 bis 0,06 dA/min. Als Standardkurve dient eine Verdünnungsreihe 1:100 bis 1:400 von Referenzplasma (Immuno AG, 1A3E). Das Prolastin (Cutter) wird aus einer 1:50-Vorverdünnung (in Aliquots bei -20°C eingefroren) 1:80 mit Tris+ verdünnt (1:4000-Verdünnung) und mit der 1:150-Verdünnung des Referenzplasmas bei jeder Meßserie mitgemessen. Bei zu starker Abweichung gegenüber der Standardgerade sollte diese Meßserie verworfen werden oder die Ergebnisse nur als ungefähre Richtwerte angesehen werden.

Die Hemmung wird errechnet nach:

$$\% = (1- (dA/min)_{Probe}/(dA/min)_{Leerwert})*100.$$

Die Hemmung der Proben soll im Bereich von 20 bis 50 % Hemmung liegen und wird dann mittels der Standardgeraden in Plasmaeinheiten umgerechnet.

**Beispiel 4: Charakterisierung verschiedener α1-AT-Produkte mittels Elastasehemmung**

Tabelle 2
Charakterisierung verschiedener A1AT -Produkte mittels Elastasehemmung und IEF

| Produkt | Protein / mg/ml | Aktivität / PE/ml | spez. Akt. / PE/mg | Reinheit | Reinigung | relative Plasma-A1AT-Aktivität |
|---|---|---|---|---|---|---|
| Plasma | 70,00 | 1,00 | 0,014 | 1,7% | 1,0 | 100,0 % |
| nach Hydroxylapatit | 1,21 | 1,22 | 1,009 | 117,8 % | 70,6 | 131,1 % |
| Serva (# 13694) | 0,44 | 0,36 | 0,814 | 95,0 % | 57,0 | 105,8 % |
| Sigma (# A-9024) | 0,49 | 0,15 | 0,299 | 34,9 % | 20,9 | n.b. |
| Prolastin (Cutter) | 41,99 | 23,95 | 0,570 | 66,6 % | 39,9 | n.b. |
| | Hauptbanden von A1AT im IEF-Gel | | | | | |
| Produkt | pI = 4,55-4,58 | pI = 4,47-4,51 | pI = 4,41-4,45 | pI = 4,35-4,39 | | |
| Plasma | + | + | + | +/- | | |
| nach Hydroxylapatit | + | + | + | + | | |
| Serva (# 13694) | + | + | + | - | | |
| Sigma (# A-9024) | + | + | + | - | | |
| Prolastin (Cutter) | + | + | + | - | | |

+/- kaum sichtbar
+ vorhanden
- nicht vorhanden
n.b. nicht berechnet

**Patentansprüche**

1. Native, chromatographisch gereinigte α1-AT-Präparation, **dadurch gekennzeichnet, dass** sie wenigstens das Isomer mit einem pI-Wert zwischen 4,3 und 4,4 enthält, eine Reinheit von wenigstens 1,0 PE/mg Protein und eine relative Plasma-α1-AT-Aktivität von mindestens 120 % aufweist.

2. Präparation nach Anspruch 1, **dadurch gekennzeichnet, dass** sie als eine pharmazeutische Präparation vorliegt.

3. Präparation nach Anspruch 1 oder 2, **dadurch gekennzeichnet, dass** sie aus einem Plasmapool gereinigt ist.

4. Präparation nach einem der Ansprüche 1 bis 3, **dadurch gekennzeichnet, dass** sie eine Reinheit von mehr als 1,1 PE/mg, vorzugsweise mehr als 1,2 PE/mg, aufweist.

5. Präparation nach einem der Ansprüche 1 bis 4, **dadurch gekennzeichnet, dass** der Anteil von inaktivem α1-AT weniger als 10 % beträgt.

6. Präparation nach Anspruch 4, **dadurch gekennzeichnet, dass** der Anteil von inaktivem α1-AT weniger als 5 %, vorzugsweise weniger als 2 %, beträgt.

7. Präparation nach einem der Ansprüche 1 bis 6, **dadurch gekennzeichnet, dass** sie frei von inaktivem α1-AT ist.

8. Präparation nach einem der Ansprüche 1 bis 7, **dadurch gekennzeichnet, dass** das α1-AT ein konservierendes ist.

9. Präparation nach einem der Ansprüche 1 bis 8, **dadurch gekennzeichnet, dass** die Isomerenverteilung des α1-AT der des nativen Proteins entspricht, insbesondere einem bei isoelektrischer Fokussierung sichtbaren Quadruplett-Bandenmuster.

10. Präparation nach einem der Ansprüche 1 bis 9, **dadurch gekennzeichnet, dass** sie zur Inaktivierung gegebenenfalls vorhandener Pathogene behandelt ist.

11. Präparation nach einem der Ansprüche 1 bis 10, **dadurch gekennzeichnet, dass** sie in einer lagerstabilen Form vorliegt, vorzugsweise als Lyophilisat oder als Lösung, insbesondere als eine Lösung, geeignet zur i.v.-Verabreichung, als Aerosol oder als Spray.

12. Präparation nach einem der Ansprüche 1 bis 11, **dadurch gekennzeichnet, dass** sie mit Liposomen, Phospholipiden bzw. anderen mikro- oder nanopartikulären Formen assoziiert vorliegt.

13. Verfahren zur Herstellung einer Präparation nach einem der Ansprüche 1 bis 12 durch Reinigung einer α1-AT enthaltenden Fraktion, erhältlich mittels Adsorptionschromatographie derart, dass α1-AT adsorbiert und aktives α1-AT, enthaltend das Isomer mit einem pI-Wert zwischen 4,3 und 4,4, in einer Fraktion durch Eluieren gewonnen wird.

14. Verfahren nach Anspruch 13, **dadurch gekennzeichnet, dass** als Ausgangsmaterial Plasma oder eine Plasmafraktion, insbesondere eine an Albumin abgereicherte Fraktion, vorzugsweise Cohn V-Niederschlag, eingesetzt wird.

15. Verfahren nach Anspruch 13 oder 14, **dadurch gekennzeichnet, dass** von einer vorgereinigten Fraktion ausgegangen wird.

16. Verfahren nach einem der Ansprüche 13 bis 15, **dadurch gekennzeichnet, dass** für die Adsorptionschromatographie ein anorganisches Chromatographiematerial, vorzugsweise Hydroxylapatit, am meisten bevorzugt keramisches Hydroxylapatit, eingesetzt wird.

17. Verfahren nach einem der Ansprüche 13 bis 16, **dadurch gekennzeichnet, dass** für die Adsorptionschromatographie ein Anionenaustauscher, bevorzugterweise in Gegenwart eines Detergens, eingesetzt wird.

18. Verfahren nach einem der Ansprüche 13 bis 16, **dadurch gekennzeichnet, dass** der Anionenaustauscher Q-Sepharose ist.

19. Verfahren nach einem der Ansprüche 13 bis 18, **dadurch gekennzeichnet, dass** die Adsorptionschromatographie unter Verwendung eines Puffers durchgeführt wird, der frei von Mercaptoethanol ist.

20. Verfahren nach einem der Ansprüche 13 bis 19, **dadurch gekennzeichnet, dass** der beim Eluieren verwendete Puffer einen pH-Wert zwischen 5,5 und 8,0 aufweist, vorzugsweise um 6, 5-6, 8.

21. Verfahren nach einem der Ansprüche 13 bis 16 und 20, **dadurch gekennzeichnet, dass** der beim Eluieren verwendete Puffer ein Salz mit einer Ionenstärke entsprechend 60 mM, vorzugsweise 40 mM, Phosphat enthält.

22. Verfahren nach einem der Ansprüche 13 bis 15 und 17 bis 20, **dadurch gekennzeichnet, dass** der beim Eluieren verwendete Puffer ein Salz mit einer Ionenstärke entsprechend 50 bis 130 mM Natriumchlorid enthält.

23. Verfahren nach einem der Ansprüche 13 bis 22, **dadurch gekennzeichnet, dass** ein weiterer Schritt ausgewählt aus der Gruppe Fällung, Filtration, Gelfiltration, Behandlung mit einem anorganischen Trägermaterial und chromatographische Reinigung durchgeführt wird.

24. Verfahren nach einem der Ansprüche 13 bis 22, **dadurch gekennzeichnet, dass** die Adsorptionschromatographie an einem Anionenaustauscher, bevorzugterweise in Gegenwart eines Detergens, mit einer Adsorption an Hydroxylapatit kombiniert wird.

**25.** Verfahren nach einem der Ansprüche 13 bis 22, **dadurch gekennzeichnet, dass** eine einzige Adsorptionschromatographie durchgeführt wird.

**26.** Verfahren nach Anspruch 25, **dadurch gekennzeichnet, dass** ein weiterer Reinigungsschritt vorgesehen ist, der keine Adsorptionschromatographie beinhaltet.

**27.** Verfahren nach einem der Ansprüche 13 bis 26, **dadurch gekennzeichnet, dass** ein Protein ausgewählt aus der Gruppe Transferrin, Albumin, Orosomucoid und Apolipoprotein in einer weiteren Fraktion gewonnen wird.

**28.** Verfahren nach einem der Ansprüche 13 bis 27, **dadurch gekennzeichnet, dass** ein Schritt zur Inaktivierung von gegebenenfalls vorhandenen Pathogenen durchgeführt wird.

**29.** Verfahren nach einem der Ansprüche 13 bis 28, **dadurch gekennzeichnet, dass** als Inaktivierung eine Behandlung mit Detergens, Lösungsmittel und/oder Hitze durchgeführt wird.

**30.** Verfahren nach einem der Ansprüche 13 bis 29, **dadurch gekennzeichnet, dass** das Eluieren derart vorgenommen wird, dass die $\alpha$1-AT-enthaltende Fraktion auch das Isomer mit einem pI-Wert zwischen 4,3 und 4,4 enthält.

**Claims**

**1.** A native, chromatographically purified $\alpha$1-AT-preparation, **characterised in that** it contains at least the isomer with a pI value of between 4.3 and 4.4, has a purity of at least 1.0 PU/mg of protein and a relative plasma $\alpha$1-AT activity of at least 120%.

**2.** A preparation according to claim 1, **characterised in that** it is provided as a pharmaceutical preparation.

**3.** A preparation according to claim 1 or 2, **characterised in that** it is purified from a plasma pool.

**4.** A preparation according to any one of claims 1 to 3, **characterised in that** it has a purity of more than 1.1 PU/mg, preferably more than 1.2 PU/mg.

**5.** A preparation according to any one of claims 1 to 4, **characterised in that** its portion of inactive $\alpha$1-AT is less than 10%.

**6.** A preparation according to claim 4, **characterised in that** its portion of inactive $\alpha$1-AT is less than 5%, preferaby less than 2%.

**7.** A preparation according to any one of claims 1 to 6, **characterised in that** it is free from inactive $\alpha$1-AT.

**8.** A preparation according to any one of claims 1 to 7, **characterised in that** the $\alpha$1-AT is a preservative one.

**9.** A preparation according to any one of claims 1 to 8, **characterised in that** the isomer distribution of the $\alpha$1-AT corresponds to that of native protein, in particular to a quadruplet band pattern visible at isoelectric focussing.

**10.** A preparation according to any one of claims 1 to 9, **characterised in that** it is treated for an inactivation of pathogens possibly present.

**11.** A preparation according to any one of claims 1 to 10, **characterised in that** it is provided in storage-stable form, preferably as a lyophilisate or as a solution, in particular as a solution suitable for i.v. administration, as an aerosol or as a spray.

**12.** A preparation according to any one of claims 1 to 11, **characterised in that** it is provided in association with liposimes, phospholipids or other micro- or nano-particulate forms.

**13.** A method of producing a preparation according to any one of claims 1 to 12 by purifying an $\alpha$1-AT-containing fraction, obtainable by means of adsorption chromatography in a manner that $\alpha$1-AT is adsorbed, and active $\alpha$1-AT containing the isomer with a pI value of between 4.3 and 4.4 is recovered in a fraction by elution.

**14.** A method according to claim 13, **characterised in that** plasma or a plasma fraction, in particular an albumin-depleted fraction, preferably Con V precipitate, is used as the starting material.

**15.** A method according to claim 13 or 14, **characterised in that** it is departed from a pre-purified fraction.

**16.** A method according to any one of claims 13 to 15, **characterised in that** an inorganic chromatographic material, preferably hydroxylapatite, most preferred ceramic hydroxylapatite, is used for the adsorption chromatography.

**17.** A method according to any one of claims 13 to 16, **characterised in that** an anion exchanger, preferably in the presence of a detergent, is used for the adsorption chromatography.

**18.** A method according to any one of claims 13 to 16, **characterised in that** the anion exchanger is Q-Sepharose.

**19.** A method according to any one of claims 13 to 18, **characterised in that** the adsorption chromatography is carried out by using a buffer which is free from mercaptoethanol.

**20.** A method according to any one of claims 13 to 19, **characterised in that** the buffer used for eluting has a pH of between 5.5 and 8.0, preferably around 6.5 and 6.8.

**21.** A method according to any one of claims 13 to 16 and 20, **characterised in that** the buffer used at elution comprises a salt having an ionic strength corresponding to 60 mM, preferably 40 mM, phosphate.

**22.** A method according to any one of claims 13 to 15 and 17 to 20, **characterised in that** the buffer used at elution comprises a salt having an anionic strength corresponding to 50 to 130 mM sodium chloride.

**23.** A method according to any one of claims 13 to 22, **characterised in that** a further step selected from the group of precipitation, filtration, gel filtration, treatment with an inorganic carrier material and chromatographic purification is carried out.

**24.** A method according to any one of claims 13 to 22, **characterised in that** the adsorption chromatography on an anion exchanger, preferably in the presence of a detergent, is combined with an adsorption on hydroxylapatite.

**25.** A method according to any one of claims 13 to 22, **characterised in that** a single adsorption chromatography is carried out.

**26.** A method according to claim 25, **characterised in that** a further purification step is provided which does not comprise an adsorption chromatography.

**27.** A method according to any one of claims 13 to 26, **characterised in that** a protein selected from the group of transferrin, albumin, orosomucoid and apolipoprotein is recovered in a further fraction.

**28.** A method according to any one of claims 13 to 27, **characterised in that** a step for inactivating pathogens possibly present is carried out.

**29.** A method according to any one of claims 13 to 28, **characterised in that** a treatment with detergent, solvent and/or heat is carried out as an inactivation.

**30.** A method according to any one of claims 13 to 29, **characterised in that** elution is carried out such that the $\alpha$1-AT-containing fraction also comprises the isomer having a pI value of between 4.3 and 4.4.

**Revendications**

**1.** Préparation d'$\alpha$1-AT native, purifiée par chromatographie, **caractérisée en ce qu'**elle comporte au moins l'isomère présentant un pI compris entre 4,3 et 4,4, une pureté d'au moins 1,0 PE/mg de protéine, et une activité de l'$\alpha$1-AT plasmatique relative d'au moins 120 %.

**2.** Préparation selon la revendication 1, **caractérisée en ce qu'**elle se présente comme une préparation pharmaceutique.

3. Préparation selon la revendication 1 ou 2, **caractérisée en ce qu'**elle est purifiée à partir d'un pool de plasma.

4. Préparation selon l'une quelconque des revendications 1 à 3, **caractérisée en ce qu'**elle présente une pureté de plus de 1,1 PE/mg, de préférence de plus de 1,2 PE/mg.

5. Préparation selon l'une quelconque des revendications 1 à 4, **caractérisée en ce que** la part d'α1-AT inactive est de moins de 10 %.

6. Préparation selon la revendication 4, **caractérisée en ce que** la part d'α1-AT inactive est de moins de 5 %, de préférence de moins de 2 %.

7. Préparation selon l'une quelconque des revendications 1 à 6, **caractérisée en ce qu'**elle est sans α1-AT inactive.

8. Préparation selon l'une quelconque des revendications 1 à 7, **caractérisée en ce que** l'α1-AT est un agent conservateur.

9. Préparation selon l'une quelconque des revendications 1 à 8, **caractérisée en ce que** la distribution des isomères de l'α1-AT correspond à celle de la protéine native, en particulier à une configuration de bandes quadruple apparente lors d'une focalisation isoélectrique.

10. Préparation selon l'une quelconque des revendications 1 à 9, **caractérisée en ce qu'**elle est traitée par l'inactivation des éventuels pathogènes présents.

11. Préparation selon l'une quelconque des revendications 1 à 10, **caractérisée en ce qu'**elle se présente sous une forme stable au stockage, de préférence comme lyophylisat ou comme solution, en particulier comme solution adaptée pour une administration en iv, comme aérosol ou comme spray.

12. Préparation selon l'une quelconque des revendications 1 à 11, **caractérisée en ce qu'**elle est associée à des liposomes, des phospholipides ou d'autres formes micro ou nanoparticulaires.

13. Procédé de réalisation d'une préparation selon l'une quelconque des revendications 1 à 12 par la purification d'une fraction contenant de l'α1-AT, pouvant être obtenue au moyen d'une chromatographie par adsorption par laquelle l'α1-AT est adsorbé et l'α1-AT actif, contenant l'isomère présentant une valeur de pI comprise entre 4,3 et 4,4 est obtenu dans une fraction par élution.

14. Procédé selon la revendication 13, **caractérisé en ce que** du plasma ou une fraction plasmatique, en particulier une fraction déplétée en albumine, de préférence un précipité V de Cohn, est utilisé comme matériau de départ.

15. Procédé selon la revendication 13 ou 14,
**caractérisé en ce qu'**il est réalisé à partir d'une fraction prépurifiée.

16. Procédé selon l'une quelconque des revendications 13 à 15, **caractérisé en ce qu'**un matériau de chromatographie anorganique est utilisé pour la chromatographie d'absorption, de préférence l'hydroxyapatite, de manière préférée entre toutes l'hydroxyapatite céramique.

17. Procédé selon l'une quelconque des revendications 13 à 16, **caractérisée en ce qu'**un échangeur d'anions est utilisé pour la chromatographie par absorption, de manière davantage préférée en présence d'un détergent.

18. Procédé selon l'une quelconque des revendications 13 à 16, **caractérisé en ce que** l'échangeur d'anion est du Q-sépharose.

19. Procédé selon l'une quelconque des revendications 13 à 18, **caractérisé en ce que** la chromatographie par absorption est réalisée avec l'utilisation d'un tampon sans mercaptoéthanol.

20. Procédé selon l'une quelconque des revendications 13 à 19, **caractérisé en ce que** le tampon utilisé pour l'élution présente un pH compris entre 5,5 et 8,0, de préférence d'environ 6,5-5,8.

**21.** Procédé selon l'une quelconque des revendications 13 à 16 et 20, **caractérisée en ce que** le tampon utilisé pour l'élution comprend un sel ayant une concentration ionique correspondant à 60 mM, de préférence 40 mM de phosphate.

**22.** Procédé selon l'une quelconque des revendications 13 à 15 et 17 à 20, **caractérisée en ce que** le tampon utilisé pour l'élution comprend un sel ayant une concentration ionique de 50 à 130 mM de chlorure de natrium.

**23.** Procédé selon l'une quelconque des revendications 13 à 22, **caractérisé en ce qu'**une étape supplémentaire choisie parmi le groupe précipitation, filtration, refiltration, traitement avec un matériau porteur anorganique et purification par chromatographie est réalisée.

**24.** Procédé selon l'une quelconque des revendications 13 à 22, **caractérisé en ce que** la chromatographie par absorption sur un échangeur d'anions, de manière davantage préférée en présence d'un détergent, est associée à une adsorption sur hydroxypalatite.

**25.** Procédé selon l'une quelconque des revendications 13 à 22, **caractérisé en ce que** une seule chromatographie par adsorption est réalisée.

**26.** Procédé selon la revendication 25, **caractérisé en ce qu'**une autre étape de purification est prévue, qui ne comprend par de chromatographie par absorption.

**27.** Procédé selon l'une quelconque des revendications 13 à 26, **caractérisé en ce qu'**une protéine choisie dans le groupe constitué par la transferrine, l'albumine, l'orosomucoïde et l'apolipoprotéine est obtenue en une fraction supplémentaire.

**28.** Procédé selon l'une quelconque des revendications 13 à 27, **caractérisé en ce qu'**une étape est réalisée pour l'inactivation des pathogènes éventuellement présents.

**29.** Procédé selon l'une quelconque des revendications 13 à 28, **caractérisé en ce qu'**un traitement avec un détergent, un milieu de dissolution et/ou à la chaleur est utilisé pour l'inactivation.

**30.** Procédé selon l'une quelconque des revendications 13 à 29, **caractérisé en ce que** l'élution est prévue de telle manière que la fraction contenant l'$\alpha$1-AT contienne aussi l'isomère présentant une valeur de pI comprise entre 4,3 et 4,4.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9535306 A
- EP 0698615 A1
- EP 0159311 A
- EP 0519901 A
- EP 0674531 A
- EP 0131740 A
- EP 0050061 A
- WO 9413329 A
- DE 4434538

**In der Beschreibung aufgeführte Nicht-Patentliteratur**

- **CARRELL et al.** *Nature,* 1982, vol. 298, 329-334
- **PATTERSON.** *Comp. Biochem. Physiol,* 1991, vol. 100 (3), 439-454
- **PAJDAK W et al.** *Folia Histochemica et Cytobiologica,* 1986, vol. 24, 169-172
- **FELDMAN ; WINKELMAN.** Blood Separation and Plasma Fractionation. Wiley-Liss, Inc, 1991, 341-383
- **BASIS et al.** *Vopr. Med. Khim,* 1987, vol. 33 (1), 54-59
- **CHAN et al.** *FEBS Lett.,* 1973, vol. 35 (1), 79-82
- **COHN.** *Review,* 1941, vol. 28, 395-417
- **J. BIETH ; B. SPIESS ; C.G. WERMUTH.** The synthesis and analytical use of a highly sensitive and convenient substrate of elastase. *Biochem. Med,* 1974, vol. 11 (4), 350-357
- **J. TRAVIS ; D. JOHNSON.** Human alpha1-Proteinase Inhibitor. *Methods of Enzymology,* 1981, vol. 80, 754-765